# EUROPEAN PATENT APPLICATION

(11) **EP 2 586 429 A1**
(43) Date of publication of application: **01.05.2013**
(21) Application number: 11186741.2
(22) Date of filing: 26.10.2011
(51) Int. Cl.: A61K 9/50

(54) **Multi-unit drug delivery device for pulsatile or sustained release**

(71) Applicant: Freund Pharmatec Ltd., Tullamore Offaly (IE)
(72) Inventor: Sivadas, Neeraj, Tullamore, County Offelay (IE); Ikeda, Masayuki, Tullamore, County Offelay (IE); Murata, Takashi, Tullamore, County Offelay (IE)
(74) Representative: Gates, Marie Christina Esther

(57) **Abstract**

The invention provides a multiparticulate timed or sustained release composition comprising a population of active ingredient-containing particles, the particles comprising a core of hydrophilic polymeric substance capable of undergoing gelation, together with an active ingredient, the core being coated with a semi-permeable film -forming polymer.

## Description

### FIELD OF THE INVENTION

The present invention relates to a novel multi-particulate dosage form that is capable of pulsatile or sustained drug delivery. The dosage form comprises an active core wherein the drug is dispersed or dissolved in a matrix consisting of a swellable protein or a polysaccharide. The active core is coated with a semi-permeable polymer to form a membrane barrier. The lag time and control of drug release from the dosage formis determined by the composition of the core and membrane as well as the thickness of the membrane barrier.

### BACKGROUND OF THE INVENTION

Drug delivery systems that provide sustained, zero order release have been the main focus of attention in the past several decades. With such systems, drug release is maintained within a therapeutic window over a prolonged period with the objective of minimising peak-to-trough fluctuations. This reduces side effects and dosage frequency, thereby improving patient compliance. However, in recent years the focus has shifted to chronotherapy and pulsatile release for which the conventional controlled/sustained drug-release systems are not ideal.

A number of factors dictated this shift in focus from a conventional sustained release approach to pulsatile delivery. A number of diseases including asthma, hypertension, angina pectoris, osteo/rheumatoid arthritis exhibit an oscillatory rhythm in their pathogenesis during a 24 hour period. These oscillations involve certain periods when there is a peak in activity with a worsening of symptoms followed by others when there's a lull. For example, in asthmatic patients, it is known that airway resistance increases progressively at night. Similarly, patients with osteoarthritis tend to have less pain in the morning and more at night; while those with rheumatoid arthritis, have pain that peaks in the morning and decreases throughout the day. With cardiovascular diseases, numerous studies have shown an increase in the incidence of early-morning myocardial infarction, sudden cardiac death or stroke. Blood pressure is at its lowest during the sleep cycle and rises steeply during the early morning awakening period (6am-noon). For effective treatment of these diseases, sustained drug release is not ideal. Instead, the release must occur after predetermined time delays (pulsatile delivery) that are in synchrony with the oscillating pattern of the disease symptoms.

Another case for pulsatile delivery is in providing site-specific drug delivery in particular regions of the gastro-intestinal tract. For example, in Inflammatory Bowel Disease, drug release only at the site of inflammation (eg. colon) is desirable. This requires that the dosage form not release any drug until it has arrived at the colon. Pulsatile delivery would also be required in the following situations: where a drug undergoes extensive first pass metabolism and therefore a fast input is required to saturate metabolising enzymes, where a sustained release of the drug leads to tolerance, i.e., a decline in the pharmacotherapeutic effect of the drug, where a drug irritates gastric mucosa or is unstable in the gastric fluid and therefore not suitable for sustained release, or where a drug has an 'absorption window' displaying high and rapid absorption in the small intestine and poor uptake in the stomach or large intestine.

The dosage forms available for pulsatile delivery can be broadly classified into `Single-unit' and 'Multiple-unit systems' as will be known to those skilled in the art. Drug release from these systems is based on the principles of swelling, erosion or dissolution of a matrix or changes in permeability or rupturing of a membrane that surrounds the active core. Multiple-unit systems are favoured over single-unit dosage forms due to benefits such as no risk of dose dumping or local irritation, predictable and reproducible gastro-intestinal transit time, increased bioavailability and the flexibility to blend different pellet types to achieve required dosage and release patterns.

The design of multiparticulate drug delivery systems has primarily involved reservoir systems with rupturable or soluble/erodible polymer coatings or systems where a change in the permeability of the coating occurs. The active core in these systems is either based on a sugar/cellulose seed onto which the drug is layered or systems where the core is manufactured using an extrusion/spheronisation technology.

US4871549 (Fujisawa Pharmaceutical Ltd) describes a Time-controlled explosion (TES) system where drug is released by explosion of the outer membrane due to the presence of an inert osmotic agent or swelling agent in the core. US5472708 (Andrx Pharmaceuticals) and US5260069 (Anda SR Pharmaceuticals) improved upon the TES system by incorporating a water soluble enteric polymer in the insoluble polymeric membrane of the TES. When the pellet reaches the elevated pH of the intestine, the polymer begins to dissolve and weakens the membrane coating, so that explosion of the weakened membrane can be assured after a predetermined time of exposure to the intestinal environment. US5840329 (BioAdvances LLC) describes a system that comprises a plurality of particles with outer coating of water insoluble, water-permeable polymer, and water-permeation adjusting agents for delivery of single or multiple precisely timed pulsed releases. US5508040 (Andrx Pharmaceuticals) describes the use of an osmotically active agent in the core (eg. sodium chloride) in addition to the drug to achieve pulsatile release. The core was then coated with a semi-permeable polymer. The lag time for drug release was dependent on the core volume and the coating thickness.

### OBJECT OF THE INVENTION

It is an object of the invention to provide a unit dosage form for the delivery of therapeutically active ingredients, that can release the active ingredient in a series of pulses or in a sustained manner. A further object is to provide a multi-particulate dosage form that delivers an active ingredient in a pulsatile fashion, i.e., a time-dependent release of drug occurring several hours after oral administration, with or without an immediate release component. A further object is to provide a multi-particulate dosage form that is capable of delivering an active ingredient in a sustained manner. A still further object is to provided a dosage unit which in addition to being capable of providing sustained release of an active ingredient, also offers the potential for pulsatile release.

### SUMMARY OF THE INVENTION

According to the present invention there is provided a multiparticulate timed or sustained release composition comprising a population of active ingredient-containing particles, the particles comprising a core of hydrophilic polymeric substance capable of undergoing gelation, together with an active ingredient, the core being coated with a semi-permeable film -forming polymer.

The gelation of the hydrophilic polymeric substance may be induced by a reduction in temperature or by a gelation-inducing agent.

The hydrophilic polymeric substance may be a protein or a polysaccharide, or a combination of both a protein and a polysaccharide. The hydrophilic polymeric substance may be selected from the group comprising gelatin, agar-agar, carrageenan, and pectin.

The particles may be spheres or cores typically of a diameter in the range of 0.5 mm to 3.0 mm, preferably 1.0mm to 2.5mm,more preferably, 1.2 mm to 1.8 mm. In other words the spheres may have a lower diameter limit of 0.5mm or 1.0mm or 1.2mm. The upper diameter limit may be 3.0mm or 2.5mm or 1.8mm.

The semi-permeable film -forming polymer forms a modified-release coating. The polymer is a water-insoluble polymeric material, but it is is water-swellable. This semi-permeability property allows water to seep in to the particles. This leads to swelling of gelatin and a build-up of internal pressure. This pressure is relieved when the coating ruptures. Rupture of the coating facilitates drug release.

The polymer may be selected from the group comprising cellulosic polymers such as ethyl cellulose, non-enteric polymethacrylates such ascellulose acetate; acrylic acid polymers and copolymers, preferably formed from acrylic acid, methacrylic acid, methyl acrylate, ethyl acrylate, methyl methacrylate and/or ethyl methacrylate, and other methacrylic resins. Particularly suitable is ethyl acrylate-methyl methacrylate-trimethylammoniumethyl methacrylate chloride terpolymer.

The semi-permeable polymeric material may further comprises a plasticizing agent. Suitable plasticizing agents include triethyl citrate, polyethylene glycol, propyleneglycol, glycerol, triacetin, dimethyl phthalate, diethyl phthalate, dibutyl phthalate, dibutyl sebacate or varying percentages ofacetylated monoglycerides. The plasticiser may be triethyl citrate.

In some embodiments the semi-permeablr polymer coating comprises a blend of two copolymers, one of which is highly water permeable and the other of which is poorly permeable.

The weight ratio of highly permeable polymer to poorly permeable polymer may be from 5:95 to 20:80, preferably 15:85 to 10:90. In other words one polymer may comprise 5 or 7 or 9 or 10 or 12 or 15 weight percent of the coating blend. The other polymer may comprise 80 or 82 or 84 or 85 or 87 or 89 or 90 weight percent of the blend.

In some embodiments, the composition may comprise at least two populations of modified release particles, the populations having different in-vitro dissolution profiles as a result of having different polymer coatings. Aletenatively the particles may have different thicknesses of polymer coating.

Thus pulses of release may be achieved by:
a) Varying the thickness of the coating membrane: This would mean that one population of the spheres would be coated to a polymer content of for example, 10% by weight and another population would be coated to a polymer content of say 15% by weight. The combination of both populations would result in a pulsed release profile.
b) Varying the permeability of the coating membrane: This would mean that one population of spheres would contain for example, a Eudragit RS:RL ratio of 85:15 and another population would contain a Eudragit RS:RL ratio of 90:10, both populations being coated to the same weight gain.
c) As will be appreciated by the person skilled in the art, a combination of approaches a and b could also be used to obtain pulses of release.

The primary idea of all three approaches (a), (b) or (c) is to control the time point at which the coating ruptures, initiating drug release.

The active ingredient may be selected from the group consisting of anticonvulsants, antidiabetic agents, anti-infective agents, analgesics, anesthetics, cardiovascular agents, central nervous system stimulants, antiParkinsonian agents, antirheumatic agents, psychotherapeutic agents, urinary tract agents or combinations thereon Specific actives may include carbachol, pilocarpine, nicotine, atropine, salmeterol, terbutaline, pseudoephedrine, terazosin, atenolol, timolol, lidocaine, procainamide, quinidine, dilitazem, verapamil, propranolol, clofibrate, fenofibrate, gemfibrozil, niacin, nicotinic acid, amlodipine, felodipine, nicardipine, nimodipine, lisinopril, captopril, valsartan, isradipine, doxazocin, amosulralol, felodipine, lercanidipine, lecidipine, nicardipine, fosinopril, imidaprile, clizapril, perindopril, losartan, irvesartan, candesartan, gliclazide, glimepiride, glipizide, beclomethasone, dexamethasone, theophylline, famotidine, nizatidine, omeprazole, pantoprazole, loperamide, mesalamine, carbamazepine, valproate sodium, alprazolam, clonazepam, diazepam, zonisamide, topiramate, codeine, fentanyl, naloxone, tramadol, tizanidine, venlafaxine, methylphenidate, chlorpromazine, fluphenazine, fluoxetine, citalopram, clomipramine, escitalopram, buspirone, doxepin, donepezil, rivastigmine, tacrine, glimipride, glipizide, metformin, sildenafil, tolterodine, tamsulosin, ibuprofen, naproxen, celecoxib, rofecoxib, piroxicam, indomethacin, alendronate, chlorpheniramine, clemastine, cefaclor, cefuroxime, vancomycin, cefepime, imipenem, clavulanic acid, ceftadizime, erythromycin, clindamycin, chloramphenicol, sulfamethoxazole, trimethoprim, isoniazid, rifamipicin, capreomycin, griseofulvin, itraconazole, ketoconazole, trimethoprim, sulfamethoxazole, praziquantel, pyrantel pamoate, gancyclovir, stavudine, zidovudine, lamivudine, indinavir, ritonavir, cisplatin, vincristine, vinblastine, docetaxel, bleomycin, doxorubicin, tamoxifen, leuprolide, zolpidem tartrate.

The composition may be adapted for oral administration The active ingredient may be dispersed or dissolved in the hydrophilic polymeric matrix, with the spheres or particlesbeing coated with a semi-permeable polymer to achieve pulsatile, delayed or sustained release of the active ingredient. The present invention thus provides a novel multi-particulate dosage form having an active core surrounded by a - semi-soluble polymeric membrane. The active core thus consists of the drug dispersed or dissolved in a swellable protein or polysaccharide based matrix.

In one embodiment of the invention, a first population of coated, drug containing spheres with delayed release properties is combined with a second population of spheres that do not have a delayed release coating to provide a two pulse dosage unit, the first pulse appearing promptly and the second pulse being delayed by the semi-permeable coating. As will be apparent to the skilled person, it would be possible to provide two or more populations of spheres with differing delayed release coatings, so that the dosage unit could provide multiple pulses of drug release at varying times. The dosage unit is thus adapted to provide a series of sequential, pulsatile releases of active ingredient. The dosage unit is thus adaptable to a variety of timing intervals of drug release, to different therapeutic agents and to combinations of agents, in order to treat a variety of conditions.

In a further embodiment of the invention, all of the active ingredient in the unit dosage form may be in a single population to provide a single pulse.

The formulation may be filled into a hard gelatin capsule, a sachet or formulated as a sprinkle.

In one embodiment the formulation is contained within a wide gauge syringe that is compatible with tube or catheter delivery.

It is well known in the art that most multiparticle systems including those described above involve the use of a non-pareil sugar seed (sugar sphere) or microcrystalline cellulose pellets (eg Cellets or Celphere). We have now developed a method to produce spheres that are made of a substance capable of undergoing gelation in which a pharmaceutically active substance can either be dissolved or dispersed. Advantageously, this system in addition to being capable of providing sustained release of an active ingredient, also offers the same potential for pulsatile release as that offered by the conventional seeds while removing the requirement for a separate drug-layering step. Drug-layering is a relatively cumbersome process that requires optimisation of multiple formulation and process related parameters (Nastruzzi et al., Influence of formulation and process parameters on pellet production by powder layering technique, AAPS PharmSciTech, 2000, 1(2), article 9).

### DESCRIPTION OF THE DRAWING

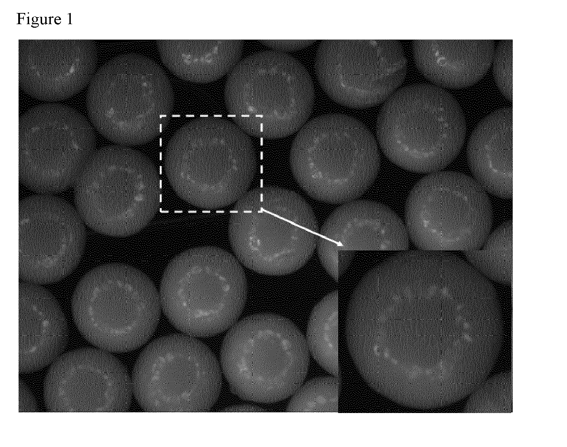

Figure 1 is an image of the nifedipine loaded minispheres coated with Eudragit RS/RL for delayed, pulsatile or sustained release of the drug in the gastrointestinal tract.

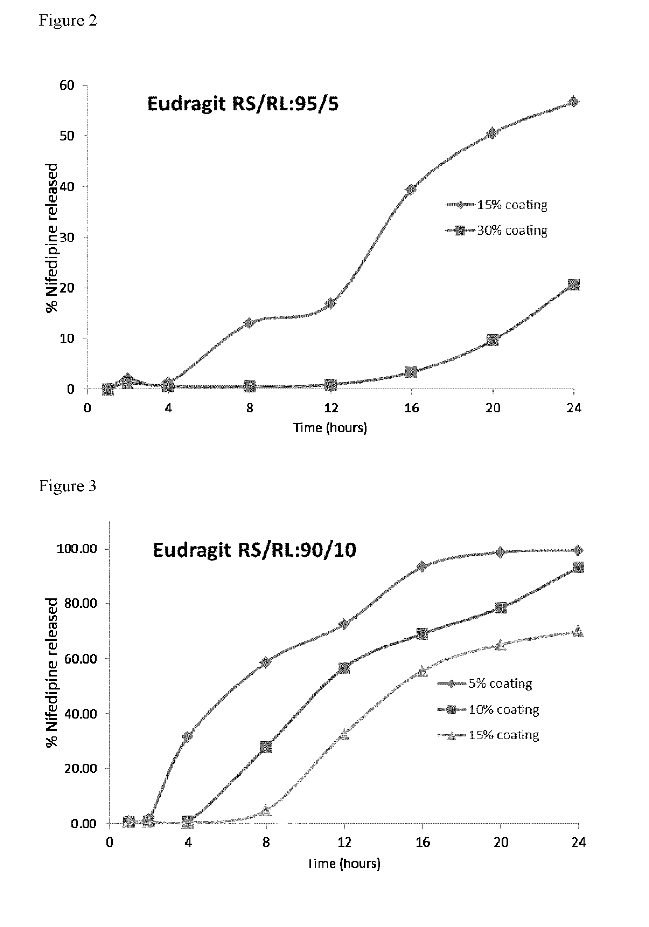

Figure 2 illustrates the dissolution profile of Nifedipine from gelatin minispheres coated with Eudragit RS/RL (95: 5 ratio) at 15 and 30% coating levels.
Figure 3 illustrates the dissolution profile of Nifedipine from gelatin minispheres coated with Eudragit RS/RL (90:10 ratio) at 5, 10 and 15% coating levels.

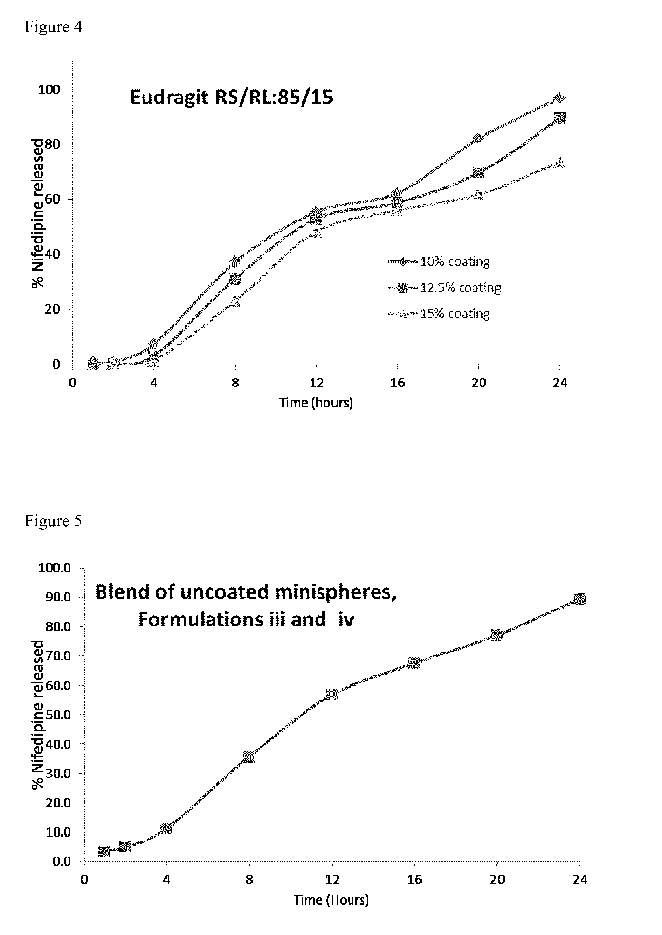

Figure 4 illustrates the dissolution profile of Nifedipine from gelatin minispheres coated with Eudragit RS/RL (85:15 ratio) at 10, 12.5 and 15% coating levels.
Figure 5 illustrates the dissolution profile of Nifedipine from a blend of gelatin minispheres containing in terms of potency, 5%w/w of uncoated spheres, 20%w/w of spheres coated with Eudragit RS/RL(90:10) at 5% coating level (Formulation iii) and 75%w/w of spheres coated with Eudragit RS/RL(90:10) at 10% coating level (Formulation iv).

### DETAILED DESCRIPTION OF THE INVENTION

The active unit of the present invention comprises a core or sphere made of a hydrophilic polymeric substance that undergoes gelation during processing of the tablet, the gelation being induced either by a lowering of temperature or by a gelation-inducing agent (eg. chloride salts such as calcium chloride, potassium chloride or magnesium chloride, calcium lactate, calcium stearate, calcium acetate, calcium gluconate and polyphosphates) ). Examples of such polymers include proteins such as gelatin or polysaccharides such as agar-agar, alginate, κ/τ-carrageenan, low methoxy pectin, chitosan, gellan, modified starches or mixtures of xanthan together with locust bean gum. Among them gelatin is preferred for the present invention.

The gelatin spheres of the present invention, incorporating the active ingredient, were manufactured according to Freund Industrial Co, Ltd. US Patent No 5,882,680 (Seamless Capsule and Method of Manufacturing the Same), the teachings of which are incorporated herein by reference. This technology is based on the principle that a laminar liquid jet can be broken into equally sized droplets by a superimposed vibration. When the droplets come in contact with a hardening liquid, they undergo gelation leading to the formation of spheres. This technique enables high-speed production of uniformly-sized spheres.

The spheres containing the active pharmaceutical ingredient may then be coated with a semi-permeable film-forming polymer to form a water-insoluble polymeric coating. A wide range of polymers can be used for the coating including, but not limited to, cellulosic polymers such as ethyl cellulose, cellulose acetate; acrylic acid polymers and copolymers, preferably formed from acrylic acid, methacrylic acid, methyl acrylate, ethyl acrylate, methyl methacrylate and/or ethyl methacrylate, and other methacrylic resins. Combinations of different coating materials or multi-layer coatings using different polymers may also be applied.

In a preferred embodiment, the semi-permeable film forming polymer is selected from those that are commercially available under the tradename Eudragite (Evonik, Darmstadt, Germany) including Eudragit® NE, RL, FS30D and RS, which are water-insoluble polymers having different degrees of permeability and expandability. The polymer solution or suspension contains the polymer(s) dissolved or suspended, respectively in a suitable aqueous or organic solvent or mixture of solvents, optionally in the presence of a lubricant. Suitable lubricants are talc, stearic acid, magnesium stearate and sodium stearate. A particularly preferred lubricant is talc. The polymer solution or suspension may optionally include a plasticizing agent. Suitable plasticizing agents include triethyl citrate, polyethylene glycol, propyleneglycol, glycerol, triacetin, dimethyl phthalate, diethyl phthalate, dibutyl phthalate, dibutyl sebacate or varying percentages of acetylated monoglycerides. A particularly preferred plasticiser is triethyl citrate.

Suitable organic solvents include isopropyl alcohol (IPA) or acetone or a mixture. The polymer solution or suspension maybe applied to the minicapsules preferably using a centrifugal coater (Freund/Vector Granurex Rotor Processor and/or CF Granulator).

The amount and type of polymer required to achieve pulsatile release can be determined by a person skilled in the art. In the present invention, the level of polymeric coating can be in the range from about 1%w/w to about 50%w/w of the batch weight of the spheres. Thus by varying the thickness of the polymeric coating, it is possible to adjust the time delay before release of the active ingredient.

In accordance with the invention the drug loaded spheres are coated with the semi-permeable and water-insoluble polymers to achieve a target release profile. Upon water ingress, drug is released from the core after rupturing of the surrounding polymer layer. This occurs due to pressure build-up within the system as a result of the swelling effect of the carrier, in this case, gelatin. As would be apparent from the examples below, the drug release in this case is time-controlled with the lag time for release from a given substrate/active core being dependent on the degree of permeability and the quantity of coating applied.

Conventionally, water soluble drugs are mainly released from a sustained/controlled release dosage form by diffusion while water insoluble drugs must undergo dissolution before release can occur. In the present invention, the mechanism of release is novel in that it is neither diffusion nor dissolution controlled but occurs as a result of a rupture or explosion of the polymeric coating that surrounds the swellable protein or polysaccharide-based active core. This mechanism of release is especially useful with water insoluble drugs in which prior art delay mechanisms that require diffusion of the drug through a permeable coating would not be effective. Advantageously, the said effect could be produced through the ready utilisation of the inherent property of certain naturally occurring proteins and polysaccharides to swell upon contact with an aqueous medium. No additional excipients such as osmotic pressure inducing agents or gas-producing effervescent agents were required to be included in the active core as is the norm in the state of the art. The use of effervescent systems with multiparticulate drug delivery systems is not desirable as it is difficult to adjust the rate and degree effervescence accurately. Effervescence is highly pH dependent and therefore affected by factors such as pH variations in the gastro-intestinal tract, intake of food etc. Drug release from osmotic core based multiparticulates has also been reported to be affected by factors such as pH and osmolality of the dissolution environment (Heinicke et al, Pharmaceutical Development and Technology, 2007, 12(5), Pg 473).

In yet another novel aspect of the invention, the use of a core made of a high-molecular weight polymer (eg. a protein or polysaccharide) makes it possible to achieve sustained drug release even after the coating that surrounds the core has ruptured. This is a result of the slow hydration, disentanglement and dissolution of the polymer chains upon contact with the aqueous environment. In conventional systems, such as those dependent on low-molecular weight sugars, eg. non-pareil sucrose beads, a structural change in the coating membrane such as a tearing or a macroscopic pore formation is accompanied by a rapid convective flow of both the drug and sucrose solution out of the device without any control being able to be exerted on the efflux rate.

### EXAMPLES

The following non-limiting examples illustrate an aspect of the invention and should not be construed to limit the scope of the invention.

### Example 1

| **Core Composition** | | |
|---|---|---|
| Ingredients | | Amount (%w/w) |
| Nifedipine (micronized) | | 8.6 |
| Glycerin | | 2 |
| Gelatin | | 18 |
| Water | | 71.4 |

To prepare the core solution, appropriate quantities of gelatin and glycerine were added to water and heated to 70°C, until in solution. Nifedipine was dispersed in this solution using a homogeniser. The spheres were then manufactured using the Spherex-Labo (SPX-Labo, Freund Corporation, Japan) using the method as described in U.S. Pat. No. 5882680. The resulting minispheres are cooled in oil for further consolidation of gelatin, centrifuged to remove residual oil and dried in a rotating drum dryer. The final spheres had an average diameter in the range 1.4-1.7mm.

The nifedipine loaded minispheres were coated with various combinations of Eudragit RS and RL using a centrifugal coater (CF-LABO, Freund Corporation). The composition of each coating system is provided in the table below. A ready-to-use solution of both polymer types in a mixure of IPA and acetone (3:2), Eudragit RS 12.5 and Eudragit RL 12.5 was used. The polymer concentration was reduced to 6.25% by adding additional IPA and acetone in the same ratio.

| Ingredients | Amount per unit dose (%w/w) | | | | | | | |
|---|---|---|---|---|---|---|---|---|
| | i | ii | iii | iv | v | vi | vii | viii |
| Eudragit RL 12.5 | 0.6 | 0.9 | 0.5 | 0.9 | 1.3 | 1.3 | 1.5 | 1.7 |
| Eudragit RS 12.5 | 10.4 | 17.6 | 4.2 | 7.9 | 11.3 | 7.6 | 8.9 | 10.1 |
| Triethyl citrate | 1.2 | 2.1 | 0.5 | 0.9 | 1.2 | 0.9 | 1.1 | 1.2 |
| Talc | 5.5 | 9.4 | 2.3 | 4.4 | 6.3 | 4.5 | 5.3 | 6.1 |

Dissolution study was done for all batches ((i) to (viii)) using the following conditions: Apparatus: Paddle at 100 rpm, Dissolution medium: 900ml of 0.1M HCl with 0.5% SLS. 5ml samples were removed at 1, 2, 4, 8, 12, 16, 20 and 24 hours. Samples were then filtered and analysed for Nifedipine content using a UV spectrophotometer set at 262nm. The data is shown in the table below and indicates that the timing of drug release can be varied by changing the composition and the thickness of the coating applied.

| Trial No. | i | ii | iii | iv | v | vi | vii | viii |
|---|---|---|---|---|---|---|---|---|
| Eudragit RS/RL ratio | 95/5 | | 90/10 | | | 85/15 | | |
| Coating level(%) | 15 | 30 | 5 | 10 | 15 | 10 | 12.5 | 15 |

| Time (Hr) | % Nifedipine released | | | | | | | |
|---|---|---|---|---|---|---|---|---|
| 1 | 0 | 0 | 0.9 | 0.5 | 0.6 | 0.8 | 0.1 | 0 |
| 2 | 2.0 | 1.1 | 1.5 | 0.8 | 0.6 | 0.9 | 0.1 | 0 |
| 4 | 1.3 | 0.6 | 31.6 | 0.7 | 0.2 | 7.2 | 2.9 | 1.3 |
| 8 | 13.0 | 0.5 | 58.6 | 27.7 | 4.8 | 37.1 | 31.1 | 23.1 |
| 12 | 16.8 | 0.8 | 72.4 | 56.6 | 32.6 | 55.4 | 52.9 | 48.1 |
| 16 | 39.4 | 3.3 | 93.3 | 69.0 | 55.6 | 62.2 | 58.7 | 55.9 |
| 20 | 50.5 | 9.6 | 98.8 | 78.4 | 65.1 | 82.0 | 69.6 | 61.6 |
| 24 | 56.6 | 20.7 | 99.5 | 93.2 | 69.9 | 96.7 | 89.3 | 73.5 |

### Example 2 Blend

| Ingredients | Amount (%w/w of potency) |
|---|---|
| Uncoated spheres | 5 |
| Coated spheres (Formulation iii) | 20 |
| Coated spheres (Formulation iv) | 75 |

The uncoated nifedipine loaded minispheres and two populations of coated minispheres (Formulations iii and iv) were blended in the ratio shown in the table above. The dissolution profile was determined as described previously in Example 1. The data is shown in the table below. A near-linear drug release pattern over a 24 hour period could be achieved.

| | Blend of 5%w/w uncoated, 20%w/w |
|---|---|
| | (Formulation iii) and 75%w/w (Formulation iv) |
| Time (Hr) | % Nifedipine released |
| 1 | 3.5 |
| 2 | 5.0 |
| 4 | 11.1 |
| 8 | 35.6 |
| 12 | 56.7 |
| 16 | 67.5 |
| 20 | 77.0 |
| 24 | 89.4 |

The words "comprises/comprising" and the words "having/including" when used herein with reference to the present invention are used to specify the presence of stated features, integers, steps or components but do not preclude the presence or addition of one or more other features, integers, steps, components or groups thereof It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

## Claims

1. A multiparticulate timed or sustained release composition comprising a population of active ingredient-containing particles, the particles comprising a core of hydrophilic polymeric substance capable of undergoing gelation, together with an active ingredient, the core being coated with a semi-permeable film -forming polymer.

2. A composition as claimed in claim 1 wherein the gelation of the hydrophilic polymeric substance may be induced by a reduction in temperature or by a gelation-inducing agent.

3. A composition as claimed in claim 1 or 2 wherein the hydrophilic polymeric substance is a protein or a polysaccharideor a combination thereof.

4. A composition as claimed in any preceding claim wherein the hydrophilic polymeric substance is water-swellable.

5. A composition according to claim 2 wherein the hydrophilic polymeric substance is selected from the group comprising gelatin, agar-agar, κ/ 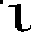 -carrageenan, and pectin, algenate, chitosan, gellan, modified starches, and mixtures of xanthan and locust bean gum.

6. A composition according to claim 1 wherein the multiparticulate minispheres are of a diameter in the range of 0.5 mm to 3.0 mm, preferably 1.0mm to 2.5mm,more preferably, 1.2 mm to 1.8 mm.

7. A composition according to claims 1 to 4 wherein the modified-release coating consists of a water-insoluble polymeric material.

8. A composition according to claim 7 wherein the semi-permeable polymeric coating is ethyl acrylate-methyl methacrylate-trimethylammoniumethyl methacrylate chloride terpolymer.

9. A composition as claimed in any preceding claim wherein the semi-permeable polymeric coatingfurther comprises a plasticizing agent.

10. A composition as claimed in claim 9 wherein the plasticising agent is triethyl citrate.

11. A composition according to claim 7 wherein at least one population of particles is coated with a polymer which is highly water permeable and another population of particles is coated with a polymer which is less water permeable.

12. A composition as claimed in claim 11 wherein the weight ratio of highly permeable polymer to less permeable polymer is from 5:95 to 20:80, preferably 15:85 to 10:90

13. A composition as claimed in any of claims 4 to 7 wherein at least one population of particles has a thicker polymer coating than another population of particles.

14. A composition as claimed in any of the preceding claims which comprises at least two populations of modified release particles, the populations having different in-vitro dissolution profiles.

15. A composition according to any of the preceding claims which is filled into a hard gelatin capsule, which is filled into a sachet the minicapsules are suspended in oil as a lubricant.which is contained within a wide gauge syringe that is compatible with tube delivery,in the form of a sprinkle.is in the form for nasal administration.
